# DEMANDE DE BREVET EUROPEEN

(11) **EP 0 576 349 A1**
(43) Date de publication de la demande: **29.12.1993**
(21) Numéro de dépôt: 93401602.3
(22) Date de dépôt: 22.06.1993
(51) Int. Cl.: C07D 209/30

(54) **Dérivés d'indolizines, procédé de préparation et utilisation pour la préparation de composés aminoalkoxybenzènesulfonyl-indolizines à activité pharmaceutique**

(30) Priorité: 23.06.1992 FR 9207663
(71) Demandeur: SANOFI, F-75008 Paris (FR)
(72) Inventeur: Gubin, Jean, B-1020 Bruxelles (BE); Renard, Michel, B-1040 Bruxelles (BE)
(74) Mandataire: Le Guen, Gérard

(57) **Abrégé**

L'invention a pour objet des dérivés d'indolizine de formule générale : 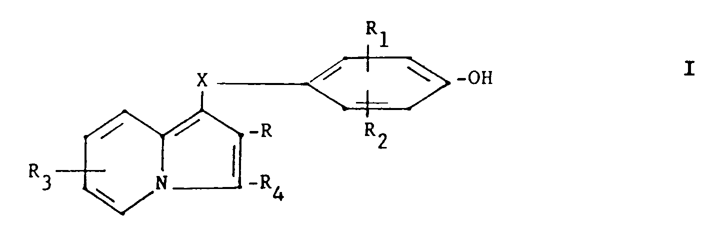
dans laquelle :
X représente un groupement -S ou -SO₂-,
R₁ et R₂, identiques ou différents, représentent chacun l'hydrogène,le radical méthyle ou éthyle ou un atome d'halogène,
R₃ représente l'hydrogène ou un radical alkyle en C₁-C₄,
R₄ représente un radical précurseur d'un groupement carboxy,
R représente un radical alkyle en C₁-C₆, cycloalkyle en C₃-C₆, ou phényle ainsi que leur utilisation en tant qu'intermédiaires pour la préparation de dérivés aminoalkoxybenzènesulfoxyl-indolizines pharmaceutiquement actifs.

## Description

La présente invention se rapporte, d'une manière générale, à de nouveaux dérivés d'indolizine, à leur procédé de préparation ainsi qu'à leur utilisation comme intermédiaires de synthèse.

Plus précisément, l'invention a pour objet les dérivés d'indolizine de formule générale : 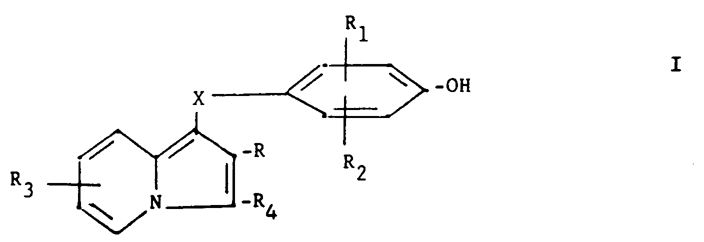
dans laquelle :
X représente un groupement -S ou -SO₂-,
R₁ et R₂, identiques ou différents, représentent chacun l'hydrogène,le radical méthyle ou éthyle ou un atome d'halogène, tel que chlore, brome ou iode,
R représente un radical alkyle en C₁-C₆, cycloalkyle en C₃-C₆ ou phényle,
R₃ représente l'hydrogène ou un radical alkyle en C₁-C₄,
R₄ représente un radical précurseur d'un groupement carboxy tel qu'un radical alkoxycarbonyle en C₁-C₄, aminocarbonyle en C₁-C₄ ou cyano.

Ainsi, en tenant compte des valeurs ci-dessus,
R peut représenter le radical méthyle, éthyle, n-propyle, isopropyle, n-butyle, n-pentyle, n-hexyle ou cyclopropyle,
R₃ peut représenter notamment le radical méthyle, éthyle, n-propyle, isopropyle ou n-butyle,
R₄ peut représenter notamment le radical méthoxycarbonyle ou éthoxycarbonyle.

Les composés de formule I dans laquelle R₁ et R₂ représentent chacun l'hydrogène, R₃ représente l'hydrogène ou le radical méthyle, R₄ représente le radical méthoxycarbonyle ou cyano et R représente le radical isopropyle ou cyclopropyle, constituent des composés préférés selon l'invention.

Les composés de formule I dans laquelle X représente le groupement -SO₂- peuvent être représentés par la formule générale : 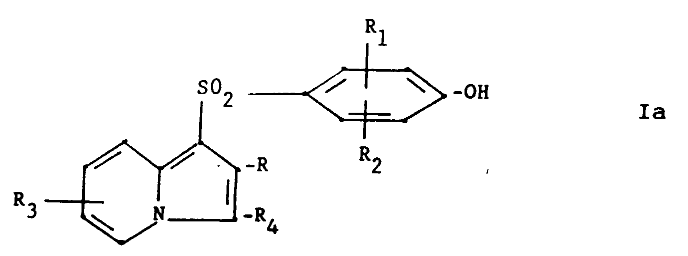
dans laquelle R, R₁, R₂, R₃ et R₄ ont la même signification que précédemment.

Ces composés se sont révélés particulièrement utiles comme produits intermédiaires, notamment pour la préparation des dérivés hydroxy-4 benzènesulfonyles de formule générale : 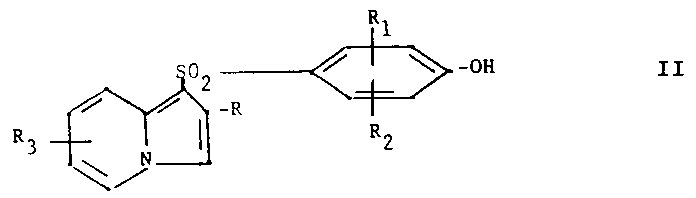
dans laquelle R, R₁, R₂ et R₃ ont la même signification que précédemment.

Ces composés de formule II peuvent être eux-mêmes largement utilisés comme intermédiaires dans la préparation de différents produits, notamment pour la synthèse finale des dérivés aminoalkoxybenzènesulfonyles de formule générale : 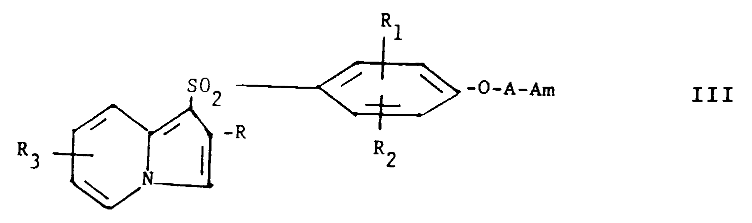
dans laquelle R, R₁, R₂, et R₃ ont la même signification que précédemment, A représente un radical alkylène en C₂-C₅ ou hydroxy-2 propylène, et Am représente un radical amino substitué, notamment :
- un radical de formule : 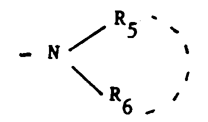 dans laquelle R₅ représente l'hydrogène ou un radical alkyle en C₁-C₈ et R₆ représente un radical alkyle en C₁-C₈ ou un radical de formule :

   -Alk -R₇

   dans laquelle Alk représente une liaison simple ou un radical alkylène en C₁-C₅ et R₇ représente un radical pyridyle, phényle, méthylènedioxy-2,3 phényle, méthylènedioxy-3,4 phényle ou un groupement phényle substitué par un ou plusieurs substituants identiques ou différents, sélectionnés parmi des atomes d'halogène, des groupements alkyle en C₁-C₄, ou des groupements alkoxy en C₁-C₄ ou R₅ et R₆, lorsqu'ils sont pris ensemble, représentent un radical alkylène ou alkénylène en C₃-C₆ éventuellement interrompu par -O-, -NH, -N= ou 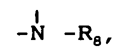 R₈ représentant un radical alkyle en C₁-C₄, phényle ou diphénylméthyle, de sorte que R₅ et R₆, pris avec l'atome d'azote auquel ils sont attachés, peuvent représenter notamment un radical pyrrolidinyle, pipéridinyle, morpholinyle, pipérazinyle, méthyl-4 pipérazinyle, phényl-4 pipérazinyle ou 1H-imidazolyle,
- un groupement de formule : 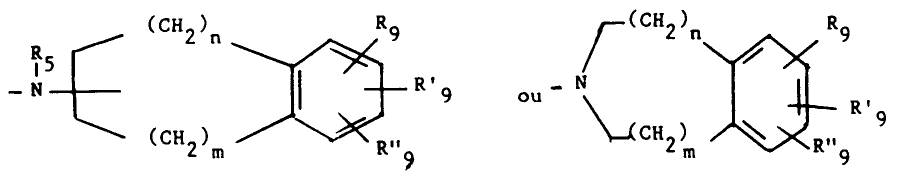 dans lequel R₅ a la même signification que précédemment, R₉, R'₉ et R''₉, qui sont identiques ou différents, représentent chacun l'hydrogène, un atome d'halogène tel que chlore ou brome, un groupement alkyle en C₁-C₄ ou alkoxy en C₁-C₄ et n et m, identiques ou différents, représentent chacun 0, 1, 2 ou 3,
   ainsi que pour la préparation de leurs sels pharmaceutiquement acceptables.

De tels dérivés aminoalkoxybenzènesulfonyles de formule III ont été décrits notamment dans les brevets ou demandes de brevet EP-A-235 111, 302 793, 382 618, 382 628 et 382 629.

Ces composés se sont révélés particulièrement intéressants pour leurs applications thérapeutiques, notamment pour leurs propriétés inhibitrices de la translocation calcique, ainsi que leurs propriétés bradycardisantes, hypotensives ou antiadrénergiques qui les rendent utiles dans le traitement de certains syndromes pathologiques du système cardiovasculaire, en particulier dans le traitement de l'angine de poitrine, de l'hypertension, de l'arythmie, de l'insuffisance circulatoire cérébrale. Dans le domaine antitumoral, ces composés sont utiles comme potentialisateurs d'anticancéreux.

Comme composés particulièrement représentatifs de cette série de dérivés aminoalkoxybenzènesulfonyles, on peut citer :
- l'isopropyl-2[{[N-méthyl N-(diméthoxy-3,4 β -phénéthyl)amino]-3 propoxy}-4 benzènesulfonyl]-1 indolizine ou fantofarone (DCI recommandée) et ses sels pharmaceutiquement acceptables,
- l'isopropyl-2 méthyl-8 [{[N- méthyl N-(diméthoxy-3,4 β-phénéthyl)amino]-3 propoxy}-4 benzènesulfonyl]-1 indolizine et ses sels pharmaceutiquement acceptables (Composé A),
- l'isopropyl-2 méthyl-8 [{[N- méthyl N-(diméthoxy-3,5 β-phénéthyl)amino]-3 propoxy}-4 benzènesulfonyl]-1 indolizine et ses sels pharmaceutiquement acceptables (Composé B),
- l'isopropyl-2 méthyl-8 {[(di-n-butylamino)-3 propoxy]-4 benzènesulfonyl}-1 indolizine et ses sels pharmaceutiquement acceptables (Composé C),
- l'isopropyl-2 [{[N- méthyl N-(triméthoxy-3,4,5 β-phénéthyl)amino]-3 propoxy}-4 benzènesulfonyl]-1 indolizine et ses sels pharmaceutiquement acceptables (Composé D),
- l'isopropyl-2 méthyl-8 {[(diméthoxy-6,7 tétrahydro-1,2,3,4 N-isoquinoléinyl-2)-3 propoxy]-4 benzènesulfonyl}-1 indolizine et ses sels pharmaceutiquement acceptables (Composé E),
- l'isopropyl-2 [{[N- méthyl N-(diméthoxy-3,4-benzyl)amino]-3 propoxy}-4 benzènesulfonyl]-1 indolizine et ses sels pharmaceutiquement acceptables (Composé F),
- l'isopropyl-2 méthyl-5 [{[N- méthyl N-(diméthoxy-3,4 β-phénéthyl)amino]-3 propoxy}-4 benzènesulfonyl]-1 indolizine et ses sels pharmaceutiquement acceptables (Composé G).

On a décrit dans le brevet EP-A-0235111 un procédé pour la préparation de dérivés de benzènesulfonyl-1 indolizine, en l'occurence des (hydroxy-4 benzènesulfonyl)-1 indolizines de formule II mettant en oeuvre les réactions suivantes :
(a) condensation de la chlorométhyl-2 pyridine avec un tosyloxy-4 benzènesulfinate de métal alcalin selon le procédé décrit dans J. Chem. Soc. 1965, p. 3090, ce qui fournit la (tosyloxy-4 benzènesulfonylméthyl)-2 pyridine,
(b) cyclisation, au reflux de ce composé avec une α-bromocétone, de manière à donner une (tosyloxy-4 benzènesulfonyl)-1 indolizine,
(c) hydrolyse de ce dérivé d'indolizine au reflux et en milieu basique de manière à produire les (hydroxy-4 benzènesulfonyl)-1 indolizines désirées.

Cette méthode présente plusieurs inconvénients, dont les plus importants peuvent être résumés comme suit :
- nécessité de préparer le tosyloxy-4 benzènesulfinate de métal alcalin selon un procédé comportant plusieurs étapes, par exemple selon un procédé en quatre étapes à partir du phénol consistant à préparer l'hydroxy-4 benzènesulfonate de métal alcalin, à tosyler ce composé, à former le chlorure de sulfonyle correspondant et finalement le sel de métal alcalin du dérivé sulfinate désiré,
- utilisation d'un excès d'α-bromocétone, produit hautement lacrymogène, souillé de produits secondaires après préparation par bromation d'une cétone,
- nécessité de mettre en oeuvre des réactions requérant un chauffage élevé et prolongé,
- manipulation de quantités importantes de produits intermédiaires en raison de la lourdeur et du volume du groupement tosyle protecteur.

La recherche d'un procédé industriel pour la préparation de dérivés d'(hydroxy-4 benzènesulfonyl)-1 indolizine de formule II mettant en oeuvre des intermédiaires de synthèse d'accès facile et peu onéreux, avec un rendement satisfaisant en produit final, reste d'un intérêt incontestable.

On connaît par ailleurs la possibilité d'induire la formation du groupement benzènesulfonyle par oxydation du groupement benzènethio. Néanmoins, cette méthode a été peu étudiée dans le cadre de l'oxydation de sulfures indoliziniques en sulfones correspondantes, probablement en raison de la dégradation bien connue du cycle indolizine sous l'effet d'agents oxydants ("Hetero-cyclic Systems with Bridgehead Nitrogen Atoms", Part One, Intersc. Publ. New York, 1961, p. 263).

Un procédé de préparation de benzènesulfonyl indolizines, à savoir des benzènesulfonyl-3 indolizines, faisant appel à une telle réaction d'oxydation du groupement benzènethio, a toutefois été rapportée dans le brevet FR-B-2.633.622.

Selon ce procédé, on oxyde, au moyen d'acide chloro-3 perbenzoïque en milieu basique, un dérivé (hydroxy-4 benzènethio)-3 indolizine O- protégé, notamment par le radical méthyle, de manière à obtenir les dérivés de benzènesulfonyl indolizine souhaités. A titre d'exemple, ce brevet français rapporte la préparation de l'isopropyl-2(méthoxy-4 benzènesulfonyl)-3 indolizine par oxydation de l'isopropyl-2(méthoxy-4 benzènethio)-3 indolizine avec un rendement de 53%.

Cette méthode se caractérise donc par la mise en oeuvre d'une étape d'oxydation d'un dérivé benzènethio comportant un radical hydroxyle protégé, tel qu'un radical méthoxy, radical hydroxyle qu'il est nécessaire de modifier dans la suite du procédé, de manière à régénérer l'hydroxyle libre.

Cette mise en oeuvre selon un couple oxydation/déprotection peut s'expliquer par le fait que la fonction hydroxyle libre est bien connue pour être sensible aux agents d'oxydation, puisque capable de s'oxyder assez facilement (Methoden der Organischen Chemie (Houben-Weyl), Band VI/1C - Phenole Teil 2 page 1121).

Dans le cadre de l'élaboration de la présente invention, on a tenté de préparer l'isopropyl-2(hydroxy-4 benzènesulfonyl)-3 indolizine au départ d'un dérivé indolizinique comportant un radical hydroxyle non protégé en l'occurence l'isopropyl-2 éthoxycarbonyl-l (hydroxy-4 benzènethio)-3 indolizine, par application d'un procédé consistant à oxyder ce dérivé hydroxy-4 benzènethio puis à déprotéger la position 1 de l'indolizine.

A cet effet, on a soumis l'isopropyl-2 éthoxycarbonyl-1 (hydroxy-4 benzènethio)-3 indolizine à l'action de l'acide chloro-3 perbenzoïque dans le N,N-diméthylformamide. Toutefois, on n'a pas enregistré la formation d'isopropyl-2 éthoxycarbonyl-1 isopropyl-2 (hydroxy-4 benzènesulfonyl)-3 indolizine attendue mais la présence, au contraire, de quantités importantes de produit de départ.

Or, on a découvert de manière surprenante, selon l'invention, qu'il est possible d'obtenir avec d'excellents rendements, des dérivés d'(hydroxy-4 benzènesulfonyl)-1 indolizine, par oxydation d'un groupement benzènethio comportant, non pas un groupement hydroxyle protégé, mais le groupement hydroxyle libre.

Selon l'invention, on prépare les dérivés sulfonyles de formule I en oxydant un composé de formule I dans laquelle X représente -S-, c'est-à-dire un dérivé benzènethio de formule générale : 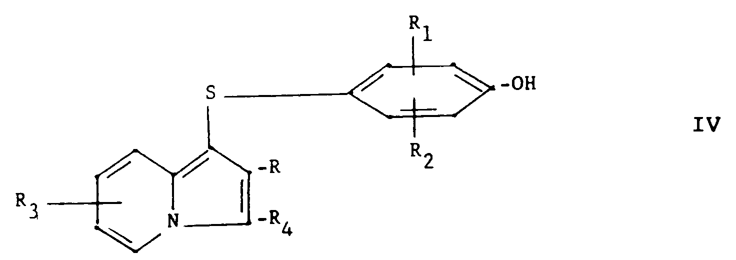
dans laquelle R, R₁, R₂, R₃ et R₄ ont la même signification que précédemment, au moyen d'un agent d'oxydation approprié tel que l'acide chloro-3 perbenzoïque ou le monoperphtalate de magnésium et dans un solvant approprié, ce qui fournit les composés désirés.

Généralement, l'oxydation se déroule à une température comprise entre -5°C et la température ambiante, de préférence à une température comprise entre 0°C et la température ambiante.

Quant au solvant, il peut s'agir d'un solvant polaire comportant un groupement amide, par exemple le N,N-diméthylformamide, le N,N-diméthylacétamide, la méthyl-2 pyrrolidone ou l'hexaméthyl phosphotriamide, un alcool en C₁-C₄, par exemple le méthanol ou l'éthanol, ou encore un nitrile tel que l'acétonitrile.

Le N,N-diméthylformamide constitue un solvant particulièrement préféré.

En général, on utilise de 2 à 2,5 équivalents d'agent oxydant, de préférence l'acide chloro-3 perbenzoïque ou le monoperphtalate de magnésium, par équivalent de composé de formule IV.

En outre, on peut envisager de tamponner le milieu réactionnel par introduction d'une base faible telle qu'un carbonate ou bicarbonate de métal alcalin ou alcalino-terreux.

Les composés de formule I dans laquelle X représente un groupement -SO₂- peuvent être synthétisés de manière très avantageuse au départ des composés de formule IV.

En effet, la formation d'une chaîne hydroxy-4 benzènesulfonyle à partir des dérivés hydroxy-4 benzènethio de formule IV peut être réalisée par mise en oeuvre d'une réaction unique, au contraire du procédé du brevet FR-B-2.633.622, qui nécessite la mise en oeuvre d'une double réaction, c'est-à-dire une oxydation d'abord, une déprotection du radical hydroxyle ensuite.

Au surplus, les composés de formule IV permettent la synthèse des composés de formule I, avec des rendements extrêmements importants, tout en évitant les inconvénients des techniques antérieures.

Par exemple, l'isopropyl-2 méthoxycarbonyl-3 (hydroxy-4 benzènesulfonyl)-1 indolizine peut être préparée à partir d'isopropyl-2 méthoxycarbonyl-3 (hydroxy-4 benzènethio)-1 indolizine, avec des rendements de 75 à 80%, tandis que la cyano-3 (hydroxy-4 benzènesulfonyl)-1 isopropyl-2 indolizine peut être obtenue au départ de cyano-3 (hydroxy-4 benzènethio)-1 isopropyl-2 indolizine avec des rendements supérieurs à 95%.

En conséquence, un autre objet de l'invention concerne les dérivés hydroxy-4 benzènethio de formule IV en tant que produits industriels nouveaux, notamment comme intermédiaires de synthèse, par exemple pour la préparation des dérivés hydroxy-4 benzènesulfonyles de formule I.

Les composés de formule IV peuvent être obtenus en faisant réagir un dérivé d'indolizine de formule générale : 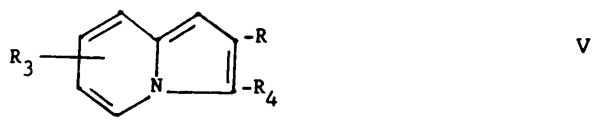
dans laquelle R, R₃ et R₄ ont la même signification que précédemment avec un composé de formule générale : 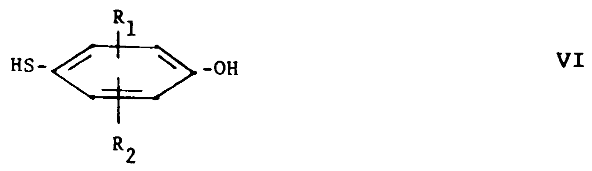
dans laquelle R₁ et R₂ ont la même signification que précédemment et ce, dans un solvant approprié tel qu'un solvant aqueux contenant un alcool en C₁-C₄, par exemple le méthanol ou un amide tel que le N,N-diméthylformamide ou l'hexaméthylphosphotriamide, en présence d'iode, et de préférence à la température de reflux du milieu réactionnel, pour obtenir les composés désirés.

Les composés de formule V et VI sont des composés connus, pouvant être obtenus selon des méthodes connues.

Par exemple, on peut obtenir les dérivés d'indolizine de formule V par mise en oeuvre des étapes suivantes :
1) réaction de la picoline-2 avec un halogénure, de préférence le bromure, de formule générale :

   Hal-CH₂-R₄ VII

   dans laquelle R₄ a la même signification que précédemment et Hal représente un atome d'halogène tel que chlore, brome ou iode, pour donner un halogénure de dérivé méthyl-2 picolinium de formule générale : 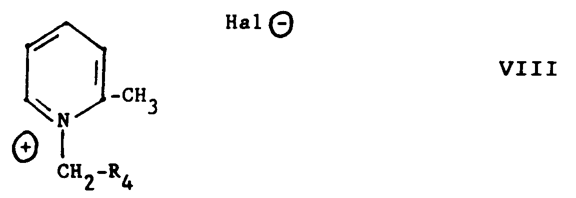 dans laquelle R₄ et Hal ont la même signification que précédemment,
2) condensation de l'halogénure du dérivé méthyl-2 picolinium en question, avec un anhydride de formule générale : 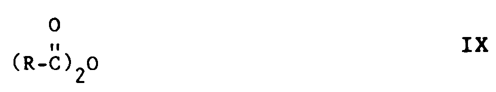 dans laquelle R a la même signification que précédemment, la réaction ayant lieu dans un solvant approprié, par exemple un hydrocarbure aromatique tel que le benzène ou le toluène, à la température de reflux du milieu et en présence d'un accepteur d'acide tel qu'une amine, par exemple la triéthylamine, ce qui fournit les composés désirés.

Comme mentionné précédemment, les dérivés d'(hydroxy-4 benzènesulfonyl) indolizine de formule Ia peuvent être utilisés pour la préparation des dérivés d'indolizine de formule II.

En conséquence, un autre objet de l'invention se rapporte à la préparation des dérivés d'indolizine de formule II par mise en oeuvre d'un procédé selon lequel
- ou bien on traite un composé de formule Ia dans un solvant approprié tel qu'un alcool, par exemple l'éthanol, à la température de reflux du milieu et en présence d'un hydroxyde de métal alcalin tel que l'hydroxyde de sodium, puis on acidifie le milieu au-moyen d'un acide fort tel que l'acide chlorhydrique ou sulfurique et décarboxyle par chauffage à une température de 130°C à 150°C, ce qui fournit les composés désirés,
- ou bien on traite un composé de formule Ia dans laquelle R₄ représente un radical cyano ou aminocarbonyle, au moyen d'un acide fort tel que l'acide sulfurique et à la température de reflux du milieu, ce qui fournit les composés désirés.

Ainsi, l'utilisation d'un composé de formule Ia dans laquelle R₄ représente notamment un groupement cyano ou aminocarbonyle permet une mise en oeuvre simplifiée, puisque selon une variante de procédé, une seule étape s'avère nécessaire pour l'élimination du groupement cyano, au contraire des composés de formule Ia dans laquelle R₄ représente un groupement alkoxycarbonyle, qui requièrent des étapes de saponification, acidification et décarboxylation.

Selon l'invention, on peut obtenir les composés de formule II avec des rendements importants, à partir du dérivé d'indolizine de formule V via les composés de formules IV et Ia.

Par exemple, l'isopropyl-2(hydroxy-4 benzènesulfonyl)-1 indolizine peut être synthétisée à partir d'isopropyl-2 méthoxycarbonyl-3 indolizine avec un rendement global de 70%.

Comme indiqué précédemment, les dérivés d'hydroxy-4 benzenesulfonyl-indolizine de formule Ia peuvent être utilisés pour la préparation des dérivés aminoalkoxybenzènesulfonyles de formule III.

En conséquence, un autre objet de l'invention concerne les dérivés hydroxy-4 benzènesulfonyles de formule Ia, en tant qu'intermédiaires pour la synthèse finale des dérivés aminoalkoxybenzènesulfonyles de formule III, en particulier pour la préparation de la fantofarone et des Composés A à G.

Par exemple, on peut préparer les composés de formule III au départ d'un dérivé hydroxy-4 benzènesulfonyle de formule II, lui-même obtenu selon l'invention à partir d'un dérivé hydroxy-4 benzènesulfonyle de formule Ia, par mise en oeuvre d'un procédé comportant la suite d'étapes suivantes :
(a) condensation du composé de formule II en présence d'un agent basique avec un dihalogénoalkane de formule générale :

   Hal-A-Hal X

   dans laquelle A et Hal ont la même signification que précédemment, et ce au reflux dans un solvant approprié, généralement un solvant polaire ou apolaire tel que la méthyl-éthyl-cétone, le N,N-diméthylformamide, le benzène, le toluène ou un xylène, ou bien,
(b1) la condensation d'un alcool halogéné de formule générale :

   Hal-A-OH XI

   dans laquelle A et Hal ont la même signification que précédemment, et ce dans un solvant tel que le N,N-diméthylformamide et en présence d'un agent basique, puis condensation du dérivé alcool obtenu avec un halogénure de formule générale :

   Hal-W XII

   dans laquelle Hal a la même signification que précédemment, et W représente un radical alkylsulfonyle en C₁-C₄, par exemple méthanesulfonyle ou arylsulfonyle en C₆-C₁₀, par exemple benzènesulfonyle ou p-toluènesulfonyle, dans un solvant accepteur d'acide, par exemple la pyridine, ou bien,
(b2) le chauffage au reflux avec une épihalohydrine telle que l'épichlorhydrine ou l'épibromydrine, en présence d'un agent basique et dans un solvant polaire tel que la méthyl-éthyl-cétone,
   pour obtenir les dérivés de sulfonyl-indolizine de formule générale : 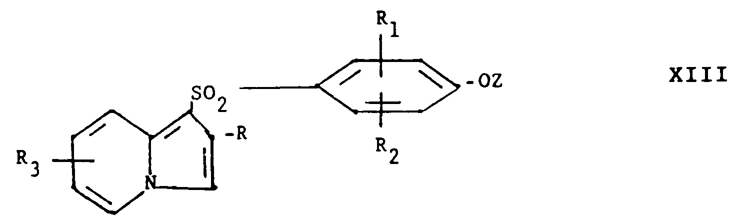 dans laquelle R, R₁, R₂ et R₃ ont la même signification que précédemment, et Z représente un radical de formule générale :

   -A-Z₁

   dans laquelle A a la même signification que précédemment, et Z₁ représente un atome d'halogène, un radical alkylsulfonyloxy en C₁-C₄ ou arylsulfonyloxy en C₆-C₁₀.

L'agent basique utilisé lors du traitement du composé de formule II est généralement un carbonate de métal alcalin, par exemple le carbonate de potassium, un hydroxyde de métal alcalin tel que l'hydroxyde de sodium ou de potassium, un hydrure de métal alcalin tel que l'hydrure de sodium ou un alcoolate de métal alcalin, par exemple le méthylate ou l'éthylate de sodium.

On fait alors réagir le dérivé de formule XIII avec une amine de formule générale :

H-Am XIV

dans laquelle Am a la même signification que précédemment, la réaction ayant lieu en présence d'un accepteur d'acide et dans un solvant approprié, généralement un solvant polaire tel qu'un alcool, par exemple le butanol, une cétone telle que la méthyl-éthyl-cétone, un hydrocarbure aromatique, par exemple le benzène, le toluène, ou un xylène, ou encore un excès d'amine de formule XIV, pour obtenir les composés de formule III sous forme de base libre, que l'on peut faire réagir, si on le désire, avec un acide approprié pour former un sel pharmaceutiquement acceptable de ce composé.

Suivant une méthode alternative, on peut également mettre en oeuvre les composés de formule II, en traitant directement un tel composé avec un halogénure de formule générale :

Hal-A-Am XV

dans laquelle Hal et Am ont la même signification que précédemment, et A représente un radical alkylène en C₂-C₅, la réaction étant conduite en présence d'un agent basique tel qu'un carbonate de métal alcalin, par exemple le carbonate de potassium, un hydroxyde de métal alcalin tel que l'hydroxyde de sodium ou de potassium, un hydrure de métal alcalin tel que l'hydrure de sodium ou un alcoolate de métal alcalin, par exemple le méthylate ou l'éthylate de sodium, pour obtenir les composés de formule III, dans laquelle A représente un radical alkylène en C₂-C₅, que l'on peut, si on le désire, faire réagir avec un acide approprié pour former un sel pharmaceutiquement acceptable de ce composé.

Les Exemples non limitatifs suivants illustrent la préparation des composés de l'invention :

### Exemple 1 :

### Préparation de l'isopropyl-2-méthoxycarbonyl-3-(hydroxy-4 benzènethio)-1 indolizine :

### a) Bromure de méthoxycarbonylméthyl-1 méthyl-2 pyridinium

Dans un ballon de 500 ml, on ajoute, à une solution de 49,3 ml (46,5 g; 0,5 mole) de picoline-2 dans 50 ml d'acétonitrile, 47,3 ml (76,5 g; 0,5 mole) de bromoacétate de méthyle. On effectue l'addition goutte à goutte et sous agitation. Après quelques minutes, la réaction devient fortement exothermique et doit être refroidie avec un bain de glace. Après trois jours sous agitation, on précipite le sel quaternaire avec un ajout d'éther éthylique sec. On filtre la poudre blanche ainsi obtenue, lave avec un peu d'éther éthylique et sèche sous vide dans un dessicateur contenant du pentoxyde de phosphore.

De cette manière, on obtient 119,93 g de bromure de méthoxycarbonylméthyl-1 méthyl-2 pyridinium.
Rendement : 98 %.
P.F. : 130°C.

### b) Isopropyl-2 méthoxycarbonyl-3 indolizine

Dans un ballon de 500 ml muni d'un réfrigérant et placé sous azote, on introduit successivement 40 g (0,162 mole) de bromure de méthoxycarbonylméthyl-1 méthyl-2 pyridinium, 80,8 ml (77,2 g; 0,487 mole) d'anhydride isobutyrique et 16 ml de toluène. On place la suspension sous agitation et on ajoute ensuite 31,2 ml (22,8 g; 0,224 mole) de triéthylamine. La suspension se colore directement en jaune. On chauffe progressivement et sous agitation jusqu'à 120°C. Après 5 heures de réaction, le milieu devenu limpide et homogène est versé dans 1 litre d'eau glacée et extrait trois fois avec 250 ml d'éther diéthylique. On lave à l'eau la phase éthérée, on la sèche sur sulfate de sodium, filtre et évapore le solvant à l'évaporateur rotatif. On distille alors le résidu sous vide important (0,03 mm Hg). La première fraction à 40°C permet de récupérer l'excès d'anhydride isobutyrique, le produit désiré distillant entre 120°C et 123°C.

De cette manière, on obtient 21,74 g d'isopropyl-2 méthoxycarbonyl-3 indolizine.
Rendement : 62%.
P.E. : 120°C - 123°C (0,03 mm Hg).
Un essai supplémentaire a permis d'obtenir un rendement de 76%.

### c) Isopropyl-2 méthoxycarbonyl-3 (hydroxy-4 benzènethio)-1 indolizine

Dans un ballon de 250 ml, placé sous azote, on dissout 5 g (0,023 mole) d'isopropyl-2 méthoxycarbonyl-3 indolizine dans un mélange de 75 ml de N,N-diméthylformamide et de 50 ml d'eau.

On place la solution sous atmosphère inerte et on injecte à la seringue 3,15 g (0,025 mole) d'hydroxy-4 benzènethiol préalablement dissous dans 25 ml de N,N-diméthylformamide. On observe une décoloration directe de l'iode. On agite alors la réaction durant 20 heures à température ambiante. On verse la solution réactionnelle dans 500 ml d'eau glacée et on extrait avec deux fois 500 ml d'éther éthylique. On lave quatre fois la phase éthérée à l'eau et une fois avec une solution de thiosulfate de sodium. On observe alors une décoloration de la phase éthérée que l'on sèche ensuite sur sulfate de sodium. On filtre et on évapore les solvants sous vide. On récupère ainsi 7,9 g d'un produit brut jaunâtre que l'on purifie par chromatographie sur colonne de silice (éluant : acétate d'éthyle/heptane 30/70) (Rf du dérivé indolizine de départ : 0,6; Rf du composé désiré : 0,3) ou par recristallisation dans l'éther diisopropylique.

De cette manière, on obtient 7,5 g d'isopropyl-2 méthoxycarbonyl-3 (hydroxy-4 benzènethio)-1 indolizine.
Rendement : 96%.
P.F. : 162°C (éther diisopropylique).

### Exemple 2 :

### Préparation de l'isopropyl-2 méthoxycarbonyl-3 (hydroxy-4 benzènesulfonyl)-1 indolizine

Dans un ballon de 250 ml, on dissout 3,41 g (0,01 mole) d'isopropyl-2 méthoxycarbonyl-3 (hydroxy-4 benzènethio)-1 indolizine dans 50 ml de N,N-diméthylformamide. On place la réaction sous agitation à 0°C (bain de glace) et on additionne alors 5,44 g (0,022 mole) d'acide chloro-3 perbenzoïque préalablement dissous dans 50 ml de N,N-diméthylformamide. On effectue l'addition goutte à goutte, très lentement (2 h 30), et on suit la réaction par chromatographie sur couche mince de silice (C.C.M.) (éluant : acétate d'éthyle/heptane : 70/30 ; Rf du dérivé d'indolizine de départ : 0,8 ; Rf du sulfoxyde intermédiaire : 0,3 ; Rf du composé désiré : 0,5). Dès l'addition de la moitié de la quantité d'acide chloro-3 perbenzoïque (25 ml), on observe la disparition du composé de départ et la formation quantitative du sulfoxyde intermédiaire. L'oxydation de ce dernier en sulfone est beaucoup plus lente, et nécessite que la réaction soit menée à température ambiante. Après la fin de l'addition (2 h 30), on laisse la réaction sous agitation et à température ambiante durant 20 heures. Après 20 heures, une C.C.M. révèle qu'il reste encore quelques traces de sulfoxyde intermédiaire. On ajoute alors encore 0,66 g (0,003 mole) d'acide chloro-3 perbenzoïque et on laisse sous agitation durant deux heures. Après ce délai, et après un dernier contrôle C.C.M., on verse la solution devenue rougeâtre dans 500 ml d'eau glacée, et on observe la précipitation du produit désiré. Après filtration et séchage du solide orange, on récupère 4,26 g de produit brut réactionnel. On le lave, à chaud, avec 100 ml d'éther diisopropylique et on filtre pour recueillir un solide blanc légèrement brunâtre qui peut être recristallisé dans du toluène.

De cette manière, on obtient 2,8 g d'isopropyl-2 méthoxycarbonyl-3 (hydroxy-4 benzènesulfonyl)-1 indolizine.
Rendement : 75%.
P.F. : 199°C (toluène).

### Exemple 3 :

### Préparation du cyano-3 (hydroxy-4 benzène-thio)-1 isopropyl-2 indolizine

### a) Chlorure de cyanométhyl-1 méthyl-2 pyridinium

Dans un ballon de 250 ml, on ajoute, goutte à goutte, à une solution de 24,6 ml (23,25 g ; 0,25 mole) de picoline-2 dans 50 ml d'acétonitrile et 16,2 ml (19,3 g ; 0,25 mole) de chloroacétonitrile à 98%. On agite ensuite durant 18 heures au reflux, puis on refroidit dans un bain de glace. On filtre le précipité et on le lave deux fois avec de l'éther dièthylique. On sèche alors le produit très hygroscopique ainsi obtenu, un dessicateur sous vide en présence de pentoxyde76 dans de phosphore.

De cette manière, on obtient 20,35 g de chlorure de cyanométhyl-l méthyl-2 pyridinium.
Rendement : 48%.

### b) Cyano-3 isopropyl-2 indolizine

Dans un ballon de 250 ml muni d'un réfrigérant et maintenu sous azote, on introduit successivement 20,35 g (0,12 mole) de chlorure de cyanométhyl-1 méthyl-2 pyridinium, 12 ml de toluène, 62 ml (59 g ; 0,36 mole) d'anhydride isobutyrique à 97%. Tout en agitant, on ajoute, goutte à goutte, de la triéthylamine. On chauffe progressivement jusqu'à 120°C (température du bain) et on maintient cette température pendant 4 heures. On verse dans de l'eau glacée et on extrait trois fois avec de l'éther diéthylique. On lave la phase éthérée à l'eau, on sèche sur sulfate de sodium anhydre et on filtre. On évapore le solvant sous vide, puis on distille l'excès d'anhydride isobutyrique (environ 45 ml). On purifie le résidu sur colonne de gel de silice (750 g) en éluant avec un mélange d'heptane et d'acétate d'éthyle 75/25.

De cette manière, on obtient 4 g de cyano-3 isopropyl-2 indolizine.
Rendement : 18%.

### c) Cyano-3-(hydroxy-4 benzènethio)-1 isopropyl-2 indolizine

Dans un ballon de 250 ml, on dissout, sous azote, 3 g (0,0163 mole) de cyano-3 isopropyl-2 indolizine dans un mélange de 50 ml de N,N-diméthylformamide et de 36 ml d'eau. On y ajoute 2,75 g (0,0196 mole) d'hydroxy-4 benzènethiol à 90% puis, goutte à goutte, une solution de 4,14 g (0,0163 mole) d'iode dans 75 ml de N,N-diméthylformamide. On observe alors une décoloration de l'iode. On agite pendant 20 heures à température ambiante, puis on verse le contenu du ballon dans de l'eau glacée.

On extrait avec un mélange d'éther diéthylique/acétate d'éthyle, puis on lave la couche organique à l'eau. On sèche sur sulfate de sodium anhydre, filtre, évapore le solvant sous vide et purifie le résidu sur colonne de gel de silice (éluant : heptane/acétate d'éthyle 60/40).

De cette manière, on recueille 3,6 g de cyano-3(hydroxy-4 benzènethio)-1 isopropyl-2 indolizine, que l'on recristallise dans le toluène.
Rendement : 72%.
P.F. : 160°C.

### Exemple 4 :

### Préparation de la cyano-3-(hydroxy-4 benzènesulfonyl)-1 isopropyl-2 indolizine

On dissout 3 g (0,0097 mole) de cyano-3 (hydroxy-4 benzènethio)-1 isopropyl-2 indolizine dans 150 ml de N,N- diméthylformamide. On place cette solution dans un bain d'eau glacée, puis on ajoute, goutte à goutte, une solution de 4,78 g (0,0194 mole) d'acide chloro-3 perbenzoïque à 70% dans 90 ml de N,N- diméthylformamide. On agite ensuite pendant 20 heures à température ambiante, puis on verse le contenu du ballon dans de l'eau glacée. On agite durant 30 minutes, on filtre le précipité formé et on le recristallise dans l'acétate d'éthyle.

De cette manière, on obtient 3,18 g de cyano-3 (hydroxy-4 benzènesulfonyl)-1 isopropyl-2 indolizine.
Rendement : 96%.
P.F. : 232°C.

### Exemple 5 :

### Préparation de l'isopropyl-2 méthoxycarbonyl-3 (hydroxy-4 benzènesulfonyl)-1 indolizine

On dissout 2 g (0,00586 mole) d'isopropyl-2 méthoxycarbonyl-3 (hydroxy-4 phénylthio)-1 indolizine dans 45 ml de N,N- diméthylformamide. On place alors le réacteur contenant le mélange dans un bain de glace, et on ajoute, goutte à goutte, une solution de 3,62 g (0,00586 mole) d'hexahydrate de monoperphtalate de magnésium à 80% (MMPP) dans 40 ml de N,N- diméthylformamide. Un contrôle par chromatographie sur couche mince de gel de silice (CCM) (éluant : heptane/acétate d'éthyle 1/1), après l'introduction du MMPP montre que le sulfure s'est transformé en sulfoxyde. Après environ 15 heures à température ambiante, on observe en CCM un mélange de sulfone et de sulfoxyde. On refroidit et on ajoute encore 0,72 g (0,00116 mole) de MMPP dans 20 ml de N,N- diméthylformamide. Après une nouvelle période de 15 heures environ à température ambiante, on verse le mélange dans de l'eau glacée et on extrait avec de l'éther diéthylique. On lave la couche organique à l'eau et sèche sur sulfate de sodium anhydre. Après évaporation du solvant sous vide, on obtient un produit huileux rouge que l'on purifie par chromatographie sur colonne de silice (éluant : heptane/acétate d'éthyle 1/1).

De cette manière, on recueille 1,4 g d'isopropyl-2 méthoxycarbonyl-3 (hydroxy-4 benzènesulfonyl)-1 indolizine.
Rendement : 64%.

Les exemples non limitatifs suivants illustrent l'utilisation des composés de l'invention.

### Exemple A :

### Préparation de l'isopropyl-2 (hydroxy-4-benzènesulfonyl)-1 indolizine

Dans un ballon de 100 ml, on dissout, à chaud, 1,12 g (0,003 mole) d'isopropyl-2 méthoxycarbonyl-3 (hydroxy-4 benzènesulfonyl)-1 indolizine dans 30 ml d'éthanol. On y ajoute 9 ml d'une solution aqueuse 1 N d'hydroxyde de sodium, et on porte le mélange au reflux pendant 12 heures. On évapore alors l'éthanol et on dissout le sel obtenu dans 250 ml d'eau glacée. On neutralise la solution avec de l'acide chlorydhydrique 1 N jusqu'à pH = 1.

L'acide intermédiaire précipite. On le récupère par filtration, et on le sèche, ce qui fournit 1,08 g d'un solide blanc. On le place dans un petit réacteur et on chauffe durant 2 heures à 140°C sous azote. On récupère une poudre légèrement verdâtre, que l'on recristallise dans le toluène.

De cette manière, on obtient 0,927 g d'isopropyl-2 (hydroxy-4 benzènesulfonyl)-1 indolizine.
Rendement : 98%.
P.F. : 182°C (toluène).

### Exemple B :

### Préparation de l'isopropyl-2 (hydroxy-4 benzènesulfonyl-1 indolizine

On chauffe durant 48 heures à la température du reflux (bain d'huile à 120°C), 0,3 g (0,0009 mole) de cyano-3 (hydroxy-4 benzènesulfonyl)-1 isopropyl-2 indolizine dans un mélange de 3 ml d'acide acétique, 3 ml d'eau et 3 ml d'acide sulfurique concentré. On dilue avec de l'eau et on extrait avec de l'acétate d'éthyle. On lave à l'eau, sèche sur sulfate de sodium anhydre et évapore le solvant sous vide.

De cette manière, on obtient 0,2 g d'isopropyl-2 (hydroxy-4 benzènesulfonyl)-1 indolizine.
Rendement : 70%.

### Exemple C :

### Préparation de l'isopropyl-2 [{[N-méthyl N-(diméthoxy-3,4 β-phénéthyl)-amino]-3 propoxy}-4 benzènesulfonyl]-1 indolizine ou fantofarone

A 0,012 mole d'isopropyl-2 (hydroxy-4 benzènesulfonyl)-1 indolizine dans 100 ml de méthyléthyl-cétone, on ajoute 0,015 mole de chloro-1 [N-méthyl N-(diméthoxy-3,4 β-phénéthyl)-amino]-3 propane et 0,018 mole de carbonate de potassium finement broyé. On porte le mélange au reflux durant 24 heures, puis on le ramène à la température ambiante. On filtre les sels inorganiques et on évapore le filtrat sous le vide de la trompe à eau. On obtient une huile que l'on purifie par chromatographie sur colonne sèche d'alumine.

De cette manière, on obtient l'isopropyl-2 [{[N-méthyl N-(diméthoxy-3,4 β-phénéthyl)-amino]-3 propoxy}-4 benzènesulfonyl]-1 indolizine.
P.F. : 82-83°C (éther diisopropylique/dichlorométane).

## Revendications

1. Dérives d'indolizine de formule générale : 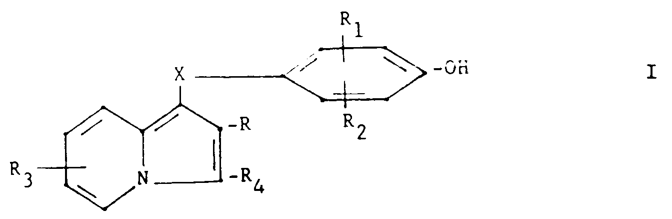 dans laquelle :
X représente un groupement -S ou -SO₂-,
R₁ et R₂, identiques ou différents, représentent chacun l'hydrogène,le radical méthyle ou éthyle ou un atome d'halogène,
R₃ représente l'hydrogène ou un radical alkyle en C₁-C₄,
R₄ représente un radical précurseur d'un groupement carboxy,
R représente un radical alkyle en C₁-C₆, cycloalkyle en C₃-C₆ ou phényle.

2. Dérivés d'indolizine selon la revendication 1, dans laquelle R₄ représente un radical alkoxycarbonyle en C₁-C₄, aminocarbonyle en C₁-C₄ ou cyano.

3. Dérivés d'indolizine selon la revendication 2, dans laquelle le radical alkoxycarbonyle en C₁-C₄ est le radical méthoxycarbonyle ou éthoxycarbonyle.

4. Dérivés d'indolizine selon l'une des revendications 1 à 3, dans laquelle R₁ et R₂ représentent chacun l'hydrogène, R₃ représente l'hydrogène ou le radical méthyle, R₄ représente le radical méthoxycarbonyle, éthoxycarbonyle ou cyano, et R représente le radical isopropyle ou cyclopropyle.

5. Isopropyl-2 méthoxycarbonyl-3 (hydroxy-4 benzènethio)-1 indolizine.

6. Isopropyl-2 méthoxycarbonyl-3 (hydroxy-4 benzènesulfonyl)-1 indolizine.

7. Isopropyl-2 cyano-3 (hydroxy-4 benzènethio)-1 indolizine.

8. Isopropyl-2 cyano-3 (hydroxy-4 benzènesulfonyl)-1 indolizine.

9. Procédé de préparation de dérivés d'indolizine selon la revendication 1, dans laquelle X représente le groupement -SO₂-, caractérisé en ce que l'on oxyde un dérivé benzènethio de formule générale : 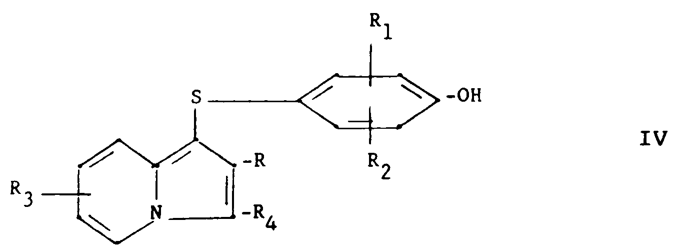 dans laquelle R, R₁, R₂, R₃ et R₄ ont la même signification que dans la revendication 1, au moyen d'un agent d'oxydation, pour obtenir les composés désirés.

10. Procédé selon la revendication 9, caractérisé en ce que l'agent d'oxydation est l'acide chloro-3 perbenzoïque ou le monoperphtalate de magnésium.

11. Procédé selon la revendication 9 ou 10, caractérisé en ce que l'on utilise de 2 à 2,5 équivalents d'agent d'oxydation par équivalent de dérivé benzènethio de formule IV.

12. Procédé selon une des revendications 9 à 11, caractérisé en ce que l'oxydation a lieu à une température comprise entre -5°C et la température ambiante.

13. Procédé de préparation de dérivés d'indolizine selon la revendication 1, dans laquelle X représente le groupement -S-, caractérisé en ce que l'on fait réagir un dérivé d'indolizine de formule générale : 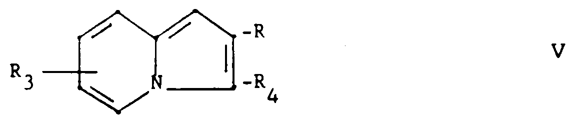 dans laquelle R, R₃ et R₄ ont la même signification que dans la revendication 1, avec un composé de formule générale : 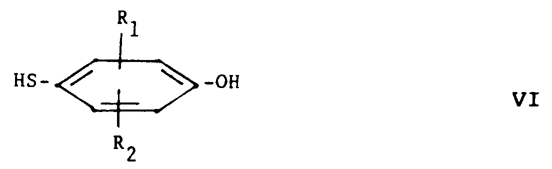 dans laquelle R₁ et R₂ ont la même signification que précédemment, et ce dans un solvant approprié, en présence d'iode et à la température de reflux, pour obtenir les composés désirés.

14. Procédé selon une des revendications 9 à 12, caractérisé en ce que le solvant est un solvant polaire.

15. Procédé selon la revendication 14, caractérisé en ce que le solvant est le N,N-diméthylformamide, l'hexaméthylphosphotriamide, l'acétonitrile ou un alcool en C₁-C₄.

16. Procédé de préparation de dérivés hydroxy-4 benzènesulfonyle de formule générale : 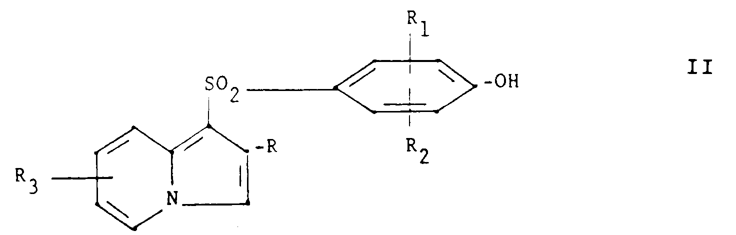 dans laquelle R, R₁, R₂, et R₃ ont la même signification que précédemment, caractérisé en ce que l'on saponifie un dérivé d'indolizine de formule générale : 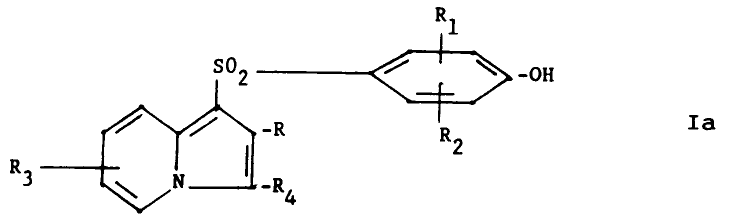 dans laquelle R, R₁, R₂, R₃ et R₄ ont la même signification que dans la revendication 1, à la température du reflux du milieu et en présence d'un hydroxyde de métal alcalin, acidifie le milieu au moyen d'un acide fort et décarboxyle par chauffage à une température de 130°C à 150°C, ce qui fournit les composés désirés.

17. Procédé de préparation de dérivés aminoalkoxybenzènesulfonyles de formule générale : 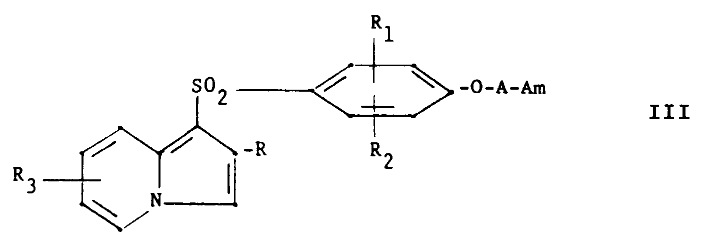 dans laquelle R, R₁, R₂, et R₃ ont la même signification que dans la revendication 1, A représente un radical alkylène en C₂-C₅ ou hydroxy-2 propylène et Am représente un radical amino substitué, caractérisé en ce que :
(a) l'on traite un dérivé d'indolizine de formule générale : 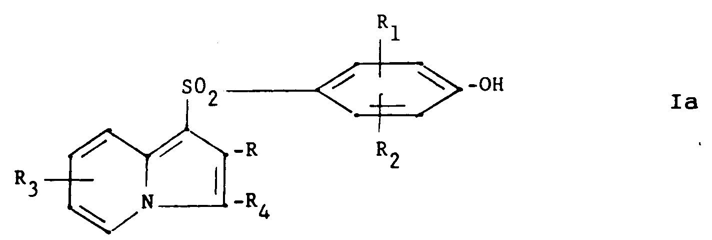 dans laquelle R, R₁, R₂, R₃ et R₄ ont la même signification que dans la revendication 1,
- ou bien avec un hydroxyde de métal alcalin à la température de reflux du milieu, acidifie le milieu au moyen d'un acide fort et décarboxyle par chauffage à une température de 130°C à 150°C,
- ou bien, lorsque R₄ représente un groupement cyano ou aminocarbonyle, avec un acide fort à la température de reflux du milieu, ce qui fournit les dérivés hydroxy-4 benzènesulfonyles de formule générale : 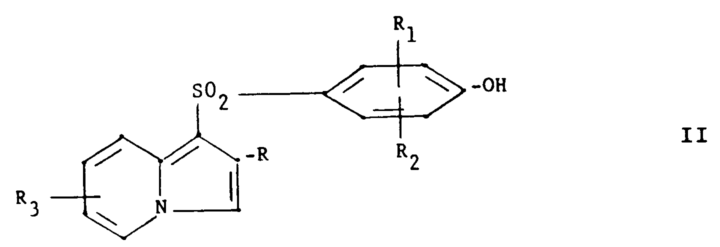 dans laquelle R, R₁, R₂, et R₃ ont la même signification que dans la revendication 1,
(b) on condense le dérivé hydroxy-4 benzènesulfonyle ainsi obtenu en présence d'un agent basique avec un dihalogénoalkane de formule générale :
Hal-A-Hal X
dans laquelle A a la même signification que précédemment et Hal représente un atome d'halogène, et ce à la température du milieu réactionnel,
- ou bien
(c 1) on condense un alcool halogéné de formule générale :
Hal-A-OH XI
dans laquelle A et Hal ont la même signification que précédemment, et ce dans un solvant et en présence d'un agent basique, puis on condense le dérivé alcool obtenu avec un halogénure de formule générale :
Hal-W XII
dans laquelle Hal a la même signification que précédemment, et W représente un radical alkylsulfonyle en C₁-C₄ ou arylsulfonyle en C₆-C₁₀, dans un solvant accepteur d'acide,
- ou bien
(c 2) on chauffe au reflux avec une épihalohydrine en présence d'un agent basique et dans un solvant polaire, pour obtenir des dérivés de sulfonyl-indolizine de formule générale : 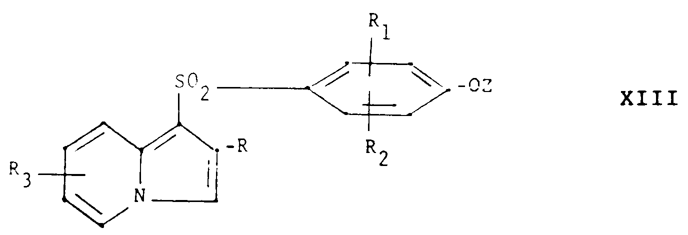 dans laquelle R, R₁, R₂ et R₃ ont la même signification que précédemment, et Z représente un radical de formule générale :
-A-Z₁
dans laquelle A a la même signification que précédemment et Z₁ représente un atome d'halogène, un radical alkylsulfonyloxy en C₁-C₄ ou arylsulfonyloxy en C₆-C₁₀, et l'on fait réagir le dérivé sulfonyl-indolizine de formule XIII avec une amine de formule générale :
H-Am XIV
dans laquelle Am a la même signification que précédemment, la réaction ayant lieu en présence d'un accepteur d'acide ou encore en présence d'un excès d'amine de formule XIV pour obtenir les composés désirés sous forme de base libre que l'on peut faire réagir, si on le désire, avec un acide approprié pour former un sel pharmaceutiquement acceptable de ce composé.

18. Procédé de préparation de dérivés aminoalkoxybenzènesulfonyles de formule générale : 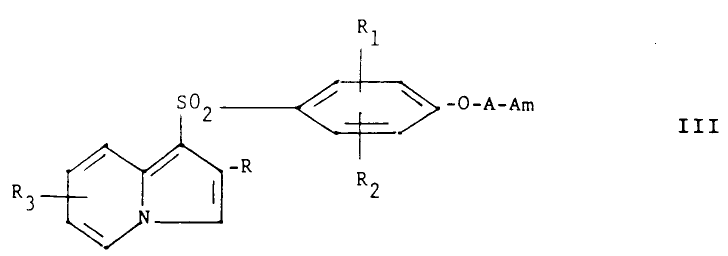 dans laquelle R, R₁, R₂ et R₃ ont la même signification que dans la revendication 1, A représente un radical alkylène en C₂-C₅ et Am représente un radical amino substitué caractérisé en ce que :
(a) l'on traite un dérivé d'indolizine de formule générale : 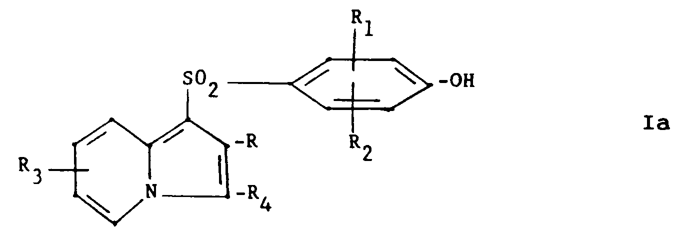 dans laquelle R, R₁, R₂, R₃ et R₄ ont la même signification que dans la revendication 1,
- ou bien avec un hydroxyde de métal alcalin, à la température de reflux du milieu, acidifie le milieu au moyen d'un acide fort et décarboxyle par chauffage à une température de 130°C à 150°C,
- ou bien, lorsque R₄ représente un groupement cyano ou aminocarbonyle au moyen d'un acide fort à la température de reflux du milieu, ce qui fournit les dérivés hydroxy-4 benzènesulfonyles de formule générale : 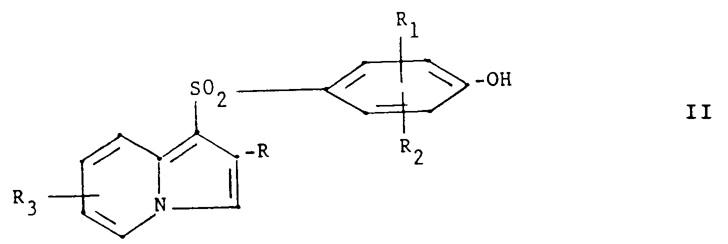 dans laquelle R, R₁, R₂, et R₃ ont la même signification que dans la revendication 1,
(b) on traite le dérivé hydroxy-4 benzènesulfonyle ainsi obtenu, avec un halogénure de formule générale :
Hal-A-Am XV
dans laquelle Am a la même signification que précédemment, Hal représente un atome d'halogène et A représente un radical alkylène en C₂-C₅, la réaction étant conduite en présence d'un agent basique, pour obtenir les composés désirés sous forme de base libre que l'on peut faire réagir, si on le désire, avec un acide approprié pour former un sel pharmaceutiquement acceptable de ce composé.

19. Procédé selon la revendication 17 ou 18, caractérisé en ce que Am représente :
- un radical de formule : 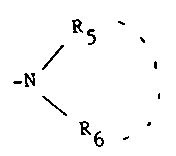 dans laquelle R₅ représente l'hydrogène ou un radical alkyle en C₁-C₈ et R₆ représente un radical alkyle en C₁-C₈ ou un radical de formule :
-Alk-R₇
dans laquelle Alk représente une liaison simple ou un radical alkylène en C₁-C₅ et R₇ représente un radical pyridyle, phényle, méthylène dioxy-2,3 phényle, méthylè- nedioxy-3,4 phényle ou un groupement phényle substitué par un ou plusieurs substituants identiques ou différents sélectionnés parmi des atomes d'halogène, des groupements alkyles en C₁-C₄ ou des groupements alkoxy en C₁-C₄ ou R₅ et R₆, lorsqu'ils sont pris ensemble, représentent un radical alkylène ou alkénylène en C₃-C₆ éventuellement interrompu par -O-, -NH-, -N= ou 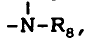 R₈ représentant un radical alkyle en C₁-C₄, phényle ou diphénylméthyle,
- un groupement de formule : 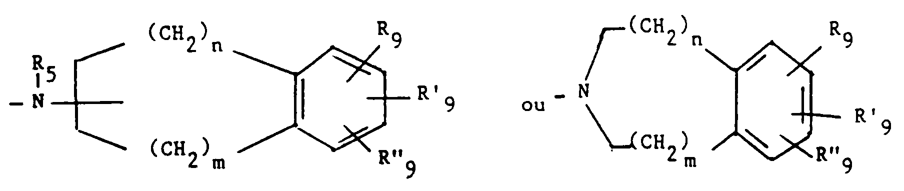 dans lequel R₅ a la même signification que précédemment, R₉, R'₉ et R''₉, qui sont identiques ou différents, représentent chacun l'hydrogène, un atome d'halogène, un groupement alkyle en C₁-C₄ ou alkoxy en C₁-C₄, et n et m, identiques ou différents, représentent chacun 0, 1, 2 ou 3.

20. Procédé selon l'une des revendications 17 à 19, caractérisé en ce que l'on prépare l'isopropyl-2 [{[N-méthyl N-(diméthoxy -3,4 β -phénéthyl)amino]-3 propoxy}-4 benzènesulfonyl]-1 indolizine et ses sels parmaceutiquement acceptables.

21. Procédé selon l'une des revendications 17 à 19, caractérisé en ce que l'on prépare l'isopropyl-2 méthyl-8 [{[N-méthyl N-(diméthoxy -3,4 β -phénéthyl)amino]-3 propoxy}-4 benzènesulfonyl]-1 indolizine et ses sels parmaceutiquement acceptables.

22. Procédé selon l'une des revendications 17 à 19, caractérisé en ce que l'on prépare l'isopropyl-2 méthyl-8 [{[N-méthyl N-(diméthoxy -3,5 β -phénéthyl)amino]-3 propoxy}-4 benzènesulfonyl]-1 indolizine et ses sels parmaceutiquement acceptables.

23. Procédé selon l'une des revendications 17 à 19, caractérisé en ce que l'on prépare l'isopropyl-2 méthyl-8 {[(di-n-butylamino)-3 propoxy]-4 benzènesulfonyl}-1 indolizine et ses sels parmaceutiquement acceptables.

24. Procédé selon l'une des revendications 17 à 19, caractérisé en ce que l'on prépare l'isopropyl-2 [{[N-méthyl N-(triméthoxy -3,4,5 β -phénéthyl)amino]-3 propoxy}-4 benzènesulfonyl]-1 indolizine et ses sels parmaceutiquement acceptables.

25. Procédé selon l'une des revendications 17 à 19, caractérisé en ce que l'on prépare l'isopropyl-2 méthyl-8 {[(diméthoxy-6,7 tétrahydro-1,2,3,4 N-isoquinoléinyl-2)-3 propoxy]-4 benzènesulfonyl}-1 indolizine et ses sels parmaceutiquement acceptables.

26. Procédé selon l'une des revendications 17 à 19, caractérisé en ce que l'on prépare l'isopropyl-2 [{[N-méthyl N-(diméthoxy-3,4 benzyl)amino]-3 propoxy}-4 benzènesulfonyl]-1 indolizine et ses sels parmaceutiquement acceptables.

27. Procédé selon l'une des revendications 17 à 19, caractérisé en ce que l'on prépare l'isopropyl-2 méthyl-5 [{[N-méthyl N-(diméthoxy-3,4 β -phénéthyl)amino]-3 propoxy}-4 benzènesulfonyl]-1 indolizine et ses sels parmaceutiquement acceptables.
